# EUROPÄISCHE PATENTSCHRIFT

(11) **EP 2 114 356 B1**
(45) Veröffentlichungstag und Bekanntmachung des Hinweises auf die Patenterteilung: **10.05.2017**
(21) Anmeldenummer: 08717409.0
(22) Anmeldetag: 05.03.2008
(51) Int. Cl.: A61K 8/04, A61K 8/92, A61Q 19/06, A61Q 19/10, C11D 1/00, C11D 7/00, A61K 8/02

(54) **HAUTREINIGUNGSMITTEL MIT PARTIKELN ENTHALTEND HYDRIERTES RIZINUSÖL**
SKIN CLEANING AGENT WITH PARTICLES CONTAINING HYDROGENATED CASTOR OIL
PRODUIT DE NETTOYAGE DE LA PEAU AVEC DES PARTICULES COMPRENANT DE L'HUILE DE RICIN HYDROGENÉE

(30) Priorität: 06.03.2007 EP 07103635
(43) Veröffentlichungstag der Anmeldung: 11.11.2009
(73) Patentinhaber: Peter Greven Hautschutz GmbH & Co. KG, 53902 Bad Münstereifel (DE)
(72) Erfinder: STOLZ, Hermann-Josef, 53902 Bad Münstereifel (DE); BÖRNICKE, Robert, 53902 Bad Münstereifel (DE); MATZEL, Manfred, 50935 Köln (DE)
(74) Vertreter: dompatent von Kreisler Selting Werner - Partnerschaft von Patent- und Rechtsanwälten mbB
(86) Internationale Anmeldenummer: PCT/EP2008/052659
(87) Internationale Veröffentlichungsnummer: WO 2008/107453

(56) Entgegenhaltungen:
- EP-A- 0 711 544
- EP-A- 1 634 565
- EP-A1- 0 692 236
- DE-A1- 2 725 924
- DE-A1- 10 059 668
- DE-A1- 10 210 449
- FR-A1- 2 737 668
- FR-A1- 2 775 441
- FR-A1- 2 885 538
- JP-A- 2004 189 612
- DISCLOSED ANONYMOUSLY: "Novel wax crystal emulsions and gels" RESEARCH DISCLOSURE, MASON PUBLICATIONS, HAMPSHIRE, GB, Bd. 511, Nr. 76, November 2006 (2006-11), Seite 1516, XP007136866 ISSN: 0374-4353
- NN: "Birken-Dusch-Peeling" WELEDA, [Online] 2007, Seiten 1-1, XP002446843 Gefunden im Internet: URL:http://www.weleda.de/Koerperpflege/Kop fbisFuss/Duschlotionen/Birkenduschpeeling. html#> [gefunden am 2007-08-13]

## Beschreibung

Die Erfindung betrifft ein Hautreinigungsmittel, einen Reinigungskörper für das Hautreinigungsmittel sowie Verfahren zur Herstellung des Reinigungskörpers.

Hautreinigungsmittel können zur Verbesserung der Reinigungsleistung Abrasiva enthalten, die die Reinigungswirkung von tensidartigen Komponenten mechanisch unterstützen.

Zahlreiche anorganische und organische Abrasiva sind bekannt, die beispielsweise in Handreinigern oder Peeling-Präparaten eingesetzt werden.

Die zuerst verwendeten Abrasiva waren auf Basis von mineralischen Komponenten wie Quarzsand oder Bimsmehl. Nachteilig hieran ist jedoch, dass Quarzsand und Bimsmehle harte, scharfkantige Körner haben, was zu einer schlechten Hautverträglichkeit führt. Aufgrund ihrer hohen Dichte neigen sie zur Sedimentation und führen damit zu einem Verstopfen der Abflüsse.

Weitere eingesetzte Komponenten sind Holzmehle. Diese haben eine verbesserte Hautverträglichkeit und sedimentieren in den Abflüssen nicht mehr. Allerdings besteht durch die Verwendung von Holzmehl grundsätzlich das Risiko von Allergien gegen Terpeninhaltsstoffe des Holzes. Darüber hinaus ist nachteilig, dass Holzmehl In Verdacht steht, krebserregend zu sein, so dass strenge Sicherheitsvorkehrungen bei der Herstellung und Verarbeitung von Holzmehl zu beachten sind.

Weitere Abrasiva sind auf das Basis von Polyurethan- oder Polyethylenmehl erhältlich. Von ihrer Hautverträglichkeit, der Härte, etc. sind sie hervorragend als Abrasiva geeignet. Allerdings sind sie praktisch biologisch nicht abbaubar, so dass sie in Bezug auf Umweltschutz und Nachhaltigkeit nicht optimal sind.

Eine weitere Gruppe von Abrasiva sind auf der Basis von Mehlen von Naturstoffen erhältlich.

Die DE 40 38 076 C2 beschreibt beispielsweise Schalen- und Kernmehle, insbesondere Walnussschalenmehle.

EP 1 106 173 A2 beschreibt den Einsatz von Maiskolbenmehlen als Abrasiva.

Hierbei ist zu bedenken, dass zum Einsatz eine Desinfektion des eingesetzten Mehles notwendig ist, um eine Verkeimung der Produkte zu vermeiden. Die oben genannte EP 1 106 173 beschreibt ein Bleichverfahren für Naturmehle, das gleichzeitig gegebenenfalls vorhandene Keime abtötet. Während grundsätzlich die Verwendung solcher Naturstoffmehle unter Nachhaltigkeitsgesichtspunkten gut geeignet ist, sind die Eigenschaften solcher Mehle weiterhin nicht optimal.

Der Grad der Verschmutzung an den verschiedenen Arbeitsplätzen in der Industrie nimmt durch den gestiegenen Anspruch an Arbeitsicherheit und -hygiene ab. Daher besteht die Forderung, nicht zuletzt von Berufsdermatologen, nach hautfreundlicheren Produkten. Im Fokus stehen dabei vor allem die Reibemittel.

US 2003/0180235 beschreibt Verfahren zur Herstellung von Mikropartikeln, wobei die Mikropartikel geringe Anteile an Rizinusöl enthalten können.

EP 0 711 544 A2 beschreibt Zahnputzzusammensetzung, die Agar umhüllte Kapseln enthalten, die mit medizinischen Wirkstoffen gefüllt sind. Ein Vergleichsbeispiel zeigt die Verwendung von Kapseln, die Rizinusöl und Natrium-Kupferchlorophylin enthalten.

DE 100 59 668 A1 beschreibt nanopartikuläre Wachse mit einem mittleren Teilchendurchmesser von 5 bis 500 nM. Aufgrund der geringen Größe wirken diese nicht als Reinigungskörper.

Es besteht immer noch ein Bedarf an Hautreinigungsmitteln, die gute Reinigungsleistungen bieten, jedoch verbesserte, hautschonende Eigenschaften aufweisen. Aufgabe der vorliegenden Erfindung war es, einen Reinigungskörper und ein Hautreinigungsmittel enthaltend den Reinigungskörper bereit zu stellen, die die genannten Nachteile des Standes der Technik überwindet, insbesondere eine hohe Hautverträglichkeit mit sich bringen.

Gelöst wird die Aufgabe durch ein Hautreinigungsmittel enthaltend
- 2 bis 25 Gew.-% eines Reinigungskörpers mit einer mittleren Korngröße von 100 bis 1.000 µm enthaltend hydriertes Rizinusöl,
- 2 bis 30 Gew.-% Tenside,
- 0,1 bis 10 Gew.-% Verdickungsmittel,
- Wasser, sowie gegebenenfalls weiteren Hilfsstoffe.

Erfindungsgemäß werden Reinigungskörper eingesetzt, die Rizinusöl, d.h. das aus *Rizinus communis* Samen gewonnene Öl, das anschließend zumindest teilweise hydriert wird, enthalten.

Der erfindungsgemäße Reinigungskörper enthält mindestens 50% Rizinusöl, bevorzugt mindestens 70% Rizinusöl und am meisten bevorzugt mindestens 90 Gew.-% Rizinusöl. Erfindungsgemäss enthält der Reinigungskörper kein Natrium-Kupferchlorophylin.

Der Grad der Hydrierung kann durch die Bestimmung der Jodzahl ermittelt werden. Besonders geeignet haben sich Rizinusöle mit einer Jodzahl von 0 bis 20 mg I₂/100g erwiesen.

Der Gehalt an Reinigungskörpern im Hautreinigungsmittel liegt bevorzugt bei mindestens 3 Gew.-%, mehr bevorzugt bei mindestens 5 Gew.-%, noch mehr bevorzugt bei mindestens 6 Gew.-% oder mindestens 7 Gew.-%. Überraschenderweise zeigen die erfindungsgemäßen Reinigungskörper ein schmutzlöse- und schmutzabsorbierendes Verhalten. Schmutzpartikel bilden beim Einsatz des erfindungsgemäßen Reinigungsmittels eine Art Umhüllung um die erfindungsgemäßen Reinigungskörper. Auf diesem Wege wird eine gute Reinigungswirkung erreicht.

Die erfindungsgemäßen Reinigungskörper zeigen keinen oder nur untergeordneten abrasiven (abschabenden) Effekt; das erfindungsgemäße Reinigungsmittel ist daher besonders hautschonend.

Grundsätzlich können die Reinigungskörper neben dem hydrierten Rizinusöl auch noch weitere Stoffe, insbesondere Wachse enthalten. Besonders geeignet sind Paraffinwachse, Carnaubawachse, Candelilawachse, Polyethylenwachse, oxidierte Polyethylenwachse oder Mischungen davon. Es ist darauf zu achten, solche Wachse zu wählen, die einen Schmelzbereich oberhalb von 40°C, bevorzugt oberhalb von 80°C aufweisen.

Überraschenderweise zeigt sich, dass die Reinigungskörper auch in Gegenwart von Lösungsmitteln wie dibasischem Ester (DBE) stabil sind. Damit können die Reinigungskörper auch in Spezialprodukten wie Lackreinigern eingesetzt werden.

Überraschenderweise zeigt sich, dass sich die eingesetzten Reinigungskörper trotz der Anwesenheit der Tenside im Hautreinigungsmittel nicht auflösen.

Überraschenderweise zeigt sich auch, dass der erfindungsgemäß eingesetzte Reinigungskörper unter den üblichen Transport- und Lagerungsbedingungen stabil bleiben.

Als Tenside zur Verwendung im Hautreinigungsmittel haben sich insbesondere nicht-ionische, zwitter-ionische und anionische Tenside als geeignet erwiesen, beispielsweise Ethersulfate, Betaine, Alkylsulfonate, Succinate, Alkypolygylcoside, Eiweißfettsäurekondensate, Polyglycolether, Seifen und Mischungen davon. Die Auswahl entsprechender Tenside ist dem Fachmann geläufig.

Als Verdickungsmittel eignen sich insbesondere Bentonite, Xanthane, Acrylate, Alginate, Celluloseether, Carrageen und Mischungen davon.

Üblicherweise kann das erfindungsgemäße Hautreinigungsmittel einen oder mehrere der folgenden üblichen Zusatzstoffe enthalten:
- Rückfetter,
- Farbstoffe,
- Parfümstoffe,
- Titandioxid,
- Puffersubstanzen,
- Konservierungsmittel,
- flüssige Parafine,
- Glycerin,
- Antioxidationsmitteln.

Durch den Gehalt an Verdickungsmitteln und den Gehalt an Reinigungskörpern lässt sich die Fließfähigkeit des Hautreinigungsmittels einstellen. Mit geringen Gehalten an Verdickungsmitteln und Reinigungskörpern werden Fließpasten erhalten. Mit relativ hohen Gehalten werden feste Reinigungspasten erhalten, wie sie beispielsweise zur Handreinigung häufig eingesetzt werden.

Überraschenderweise ist das erfindungsgemäße Produkt sehr hautfreundlich, da das eingesetzte Rizinusöl zusätzlich Eigenschaften als Rückfetter und Emulgator besitzt.

Bevorzugt liegt das Produkt als wässrige Suspension vor. Eine Öl-in-Wasser Emulsion ist weniger bevorzugt.

Die Reinigungswirkung der Reinigungskörper kann durch die Korngröße und das Herstellverfahren beeinflusst werden. Grundsätzlich geben größere Reinigungskörper eine stärkere Wirkung.

Hergestellt wird der erfindungsgemäß Reinigungskörper bevorzugt durch ein Verfahren, bei dem das hydrierte Rizinusöl, gegebenenfalls unter Zusatz weiterer Stoffe, insbesondere weiterer Wachse, geschmolzen wird und anschließend die Schmelze vertropft oder versprüht wird.

Beim Versprühen wird die geschmolzene Wachsmischung mit hohem Druck über eine Düsen versprüht. Die Kornverteilungskurve weist eine relativ breite Basis auf. Ferner sind die Tropfen durch Zusammenschmelzen ungleichmäßiger geformt.

Vertropfte Produkte weisen eine wesentlich engere Kornverteilungskurve auf. Die Produkte sind gleichmäßig rund und neigen wegen des weiteren Tropfenkegels nicht so stark zum Verkleben. Vertropfungseinrichtungen verwenden an Stelle der Sprühdüsen eine rotierende Scheibe. Auf dieser befinden sich definierte Bohrungen, die den Durchmesser der Teilchen bestimmen.

Die unregelmäßigen Körper haben bessere mechanische Reinigungseigenschaften, jedoch dadurch bedingt eine schlechtere Hautverträglichkeit.

Grundsätzlich ist es auch möglich, die erfindungsgemäßen Reinigungskörper auf Basis von hydriertem Rizinusöl mit Abrasivstoffen, beispielsweise mit Polyurethanreibkörpern zu mischen und in Hautreinigungsmitteln einzusetzen.

Gegenstand der Erfindung ist auch ein Reinigungskörper für kosmetische Präparate mit einer mittleren Korngröße von 100 bis 1.000 µm, die hydriertes Rizinusöl enthalten sowie ein Verfahren zur Herstellung der Reinigungskörper umfassend die Schritte des Schmelzens von hydriertem Rizinusöl, gegebenenfalls zusammen mit weiteren Substanzen, gefolgt von einem Vertropfen oder Versprühen der erhaltenen Schmelze.

Gegenstand ist weiterhin die Verwendung von Reinigungskörpern mit einer mittleren Korngröße von 100 bis 1.000 µm, die hydriertes Rizinusöl enthalten, zur Herstellung von Kosmetika, insbesondere Hautreinigungsmitteln.

Die Wachse können grundsätzlich auch kosmetische Wirkstoffe enthalten. Durch eine Kombination verschiedener Wachse lassen sich die Eigenschaften der Reinigungskörper gut einstellen.

| Wachs | Tropfpunkt [°C] DIN 51801 | Penetration [0,1 mm] DIN 51579 |
|---|---|---|
| Carnaubawachs | 81 - 86 | < 1 |
| Candelillawachs | 68 - 73 | < 2 |
| Paraffinwachs (hart) | 105 - 120 | 1 - 4 |
| Paraffinwachs | 56 - 60 | 160 - 210 |
| Polyethylenwachs | 105 - 115 | 2 - 5 |
| Oxidiertes Polyethylenwachs | 101 - 109 | < 6 |
| Hydriertes Rizinus öl | 84 - 88 | 3 - 6 |

Erfindungsgemäße Reinigungskörper lassen sich auch erhalten, wenn statt des hydrierten oder teilweise hydrierten Rizinusöls ein Wachs eingesetzt wird, das ähnliche Eigenschaften hat, wie hydriertes Rizinusöl, insbesondere im Bereich der Härte und des Schmelzbereichs.

Bevorzugt ist die Abweichung bei Tropfpunkt und Penetration anderer Wachse nicht größer als 20% von den erfindungsgemäß einsetzbaren hydrierten Rizinusöl.
Figur 1 zeigt Reinigungskörper, wie sie aus dem Sprühverfahren erhalten werden.
Figur 2 zeigt Reinigungskörper, wie sie aus Vertropfungsverfahren erhalten werden.
Figur 3 zeigt die Korngrößenverteilung der Reinigungskörper gemäß Beispiel 1.
Figur 4 zeigt einen Vergleich der Abrasionswirkung verschiedener Reinigungsmittel.
Figuren 5a und 5b zeigen lichtmikroskopische Aufnahmen der Reinigungskörper vor und nach dem Kontakt mit Schmutz.

Die Erfindung wird durch die nachfolgenden Beispiele näher erläutert.

### Beispiel 1

Ein Hautreinigungsmittel mit folgender Zusammensetzung:

| INCI EU | Anteil in % |
|---|---|
| Sodium C12-18 Alkylsulfate | 15,000000 |
| Aqua | 15,000000 |
| Paraffinum Liquidum | 18,000000 |
| Aqua | 16,500000 |
| Sodium C12-15 Pareth-Sulfate | 8,750000 |
| Aqua | 3,750000 |
| Hydrogenated Castor Oil | 10,000000 |
| Aqua | 3,000000 |
| Sodium Xylenesulfonate | 2,000000 |
| Glycerin | 3,00000 |
| Oleic Acid | 1,500000 |
| Sodium Chloride | 1,200000 |
| Coco-Glucoside | 0,330000 |
| Clyceryl Oleate | 0,330000 |
| Aqua | 0,340000 |
| Phenoxyethanol | 0,560000 |
| Butylparaben | 0,060000 |
| Ethylparaben | 0,060000 |
| Methylparaben | 0,060000 |
| Propylparaben | 0,060000 |
| Titanium Dioxide | 0,200000 |
| Parfüm | 0,200000 |
| Citric Acid | 0,100000 |

### wurde hergestellt.

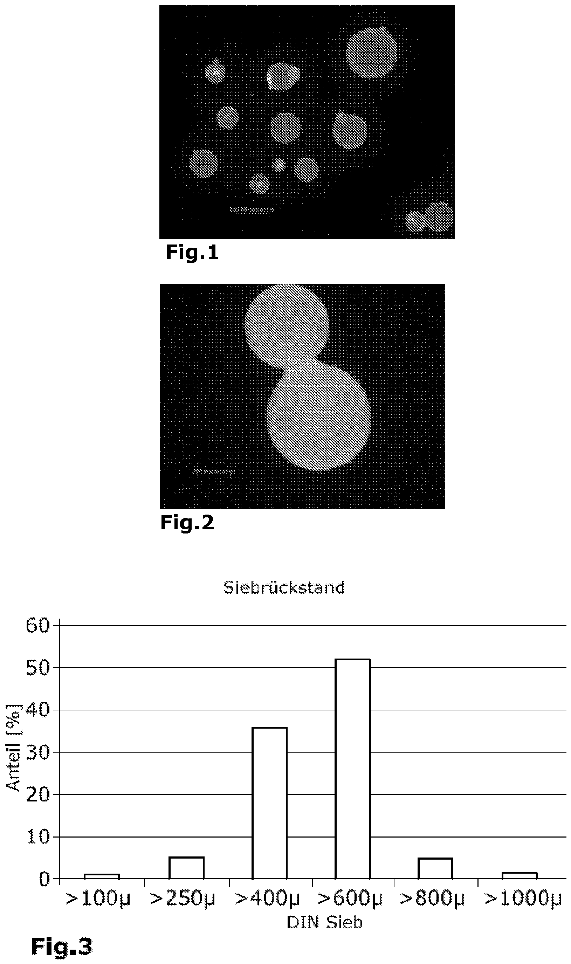

Das eingesetzte hydrierte Rizinusöl hatte eine Korngrößenverteilung gemäß Figur 3.

Es ergab sich eine relativ feste Hautreinigungspaste, die zur Entfernung starker Verschmutzung, wie Russ, Fette, Schmieröle geeignet ist. Es zeigte sich, dass die Reinigungskörper über einen längeren Zeitraum, auch unter erhöhten Temperaturbedingungen, wie sie beispielsweise bei Transport oder Lagerung im Sommer auftreten, stabil bleiben.

### Beispiel 2

| INCI EU | Anteli in % |
|---|---|
| AQUA | 60,5065 |
| SODIUM C12-15 PARETH SULFATE | 8,00 |
| HYDROGENATED CASTOR OIL (wie Beispiel 1) | 18,00 |
| SODIUM C12-18 ALKYL SULFATE | 5,00 |
| POTASSIUM COCOYL HYDROLYZED COLLAGEN | 1,00 |
| GLYCERYL RICINOLEATE | 2,00 |
| PEG-7 GLYCERYL COCOATE | 2,00 |
| BENTONITE | 2,50 |
| TITANIUM DIOXIDE | 0,60 |
| CITRIC ACID | 0,25 |
| PARFUM | 0,10 |
| 2-BROMO-2-NITROPROPANE-1,3-DIOL | 0,036 |
| CI 13015 | 0,002500 |
| CI 15510 | 0,002500 |
| CI 61570 | 0,002500 |

Das Beispiel 2 ist eine spenderfähige Fließpaste und kommt für den Bereich gröbster Verschmutzungen in Betracht. Die wirkungsvolle Tensidkombination wird durch die Verwendung von Eiweissfettsäurekondensate insgesamt wesentlich hautverträglicher. Die Reinigungswirkung wird durch das Reibemittel, das gerade für Schmierfette und -öle eine hohe Schmutzbindung aufweist, unterstützt. Rückfettende Substanzen mindern das Risiko von Hautirritationen.

### Beispiel 3

| INCI EU | Anteil in % |
|---|---|
| AQUA | 71,166 |
| SODIUM LAURETH SULFATE | 14,28 |
| Wachsmischung: | 5 |
| 85% Hydrogenated Castor oil | |
| 12% Paraffine | |
| 3% Carnauba wax | |
| COCAMIDOPROPYLBETAINE | 4,45 |
| PEG-7 GLYCERYL COCOATE | 2,00 |
| BENTONITE | 1,80 |
| CITRIC ACID | 0,55 |
| TITANIUM DIOXIDE | 0,50 |
| PARFUM | 0,10 |
| 2-BROMO-2-NITROPROPANE-1,3-DIOL | 0,054 |
| XANTHAN GUM | 0,10 |

Die Mischung hat einen Tropfpunkt von 78°C und eine Penetration von 214 bei 0,1 mm.

Das Beispiel 3 handelt es sich ebenfalls um eine Fließpaste, die bei einem mittleren Verschmutzungsgrad der Haut verwendet wird. Die Tensidkombination ist in ihrer Reinigungskraft gegenüber Beispiel 2 deutlich abgestuft. PEG-7 GLYCERYL COCOATE hat neben seinen exzellente Emulgier- und Schaumeigenschaften auch noch eine rückfettende Wirkung. Der pH-Wert ist dem der Haut angepasst.

### Beispiel 4

| INCI EU | Anteil in % |
|---|---|
| AQUA | 27,002 |
| HYDROGENATED CASTOR OIL (wie Beispiel 1) | 25,00 |
| SODIUM C12-18 ALKYL SULFATE | 23,73 |
| PARAFFINUM LIQUIDUM | 18,00 |
| POTASSIUM COCOYL HYDROLYZED COLLAGEN | 2,00 |
| CITRIC ACID | 2,00 |
| ISODECETH-7 | 1,00 |
| OLEIC ACID | 1,00 |
| PARFUM | 0,20 |
| 2-BROMO-2-NITROPROPANE-1,3-DIOL | 0,018 |
| TITANIUM DIOXIDE | 0,050 |

Das Beispiel 4 stellt eine feste bis pastenartige Handreinigungscreme dar. Sie ist für die Entfernung gröbster Verschmutzungen, wie Ruß, Schmierfette, Bitumen etc konzipiert. Die hohe Reinigungskraft begründet sich zum einen durch den Gehalt an Paraffinöl (DAB), zum anderen durch die wirkungsvolle Tensidkombination. Trotz des relativ hohen Anteils an Paraffin bleibt das Reinigungsmittel über Monate hin stabil.

### Beispiel 5

| INCI EU | Anteil in % |
|---|---|
| DIMETHYL GLUTARATE | 27,0 |
| DIMETHYL SUCCINATE | 8,3 |
| DIMETHYL ADIPATE | 6,2 |
| AQUA | 11,0 |
| DISODIUM LAURETH SULFOSUCCINATE | 9,2 |
| AQUA | 13,2 |
| HYDROGENATED CASTOR OIL (wie Beispiel 1) | 11,5 |
| ETHYLHEXYL STEARATE | 4,6 |
| PARAFFINUM LIQUIDUM | 4,6 |
| STEARALKONIUM BENTONITE | 3,0 |
| BENTONITE | 1,0 |
| PARFUM | 0,2 |
| TITANIUM DIOXIDE | 0,2 |
| | 100 |

Die Beispielrezeptur 5 gibt ein pastöses Spezialhandreinigungsmittel für die Entfernung stark haftender Verschmutzungen wie Bitumen, Lacke oder Klebstoffe wieder. Dieser starke Reinigungseffekt wird durch die Verwendung eines dibasischen Esters erreicht. Als hautverträgliches Tensid kommt ein Succinat zum Einsatz. Trotz dieses Inhaltstoffes bleibt das Reinigungsmittel stabil.

### Beispiel 6

Die Abrasionswirkung der erfindungsgemäßen Reinigungskörper wurde analysiert. Hierzu wurde als Prüfverfahren das Miller-Verfahren, genormt als ASTM-G75-01 angewendet. Dieses Verfahren ermöglicht eine standardisierte Messung der Abrasivität von partikelhaltigen Fluidfeststoffgemischen. Statt des in der Norm eingesetzten metallischen Normprüferkörper wurde ein Block aus LDPE Kunststoff eingesetzt. Die Prüfzeit wurde auf 10 min festgesetzt. Um eine pastöse Masse zu erhalten, wohin die Putzkörper im Verhältnis 1:1 mit Wasser aufgeschlemmt werden.

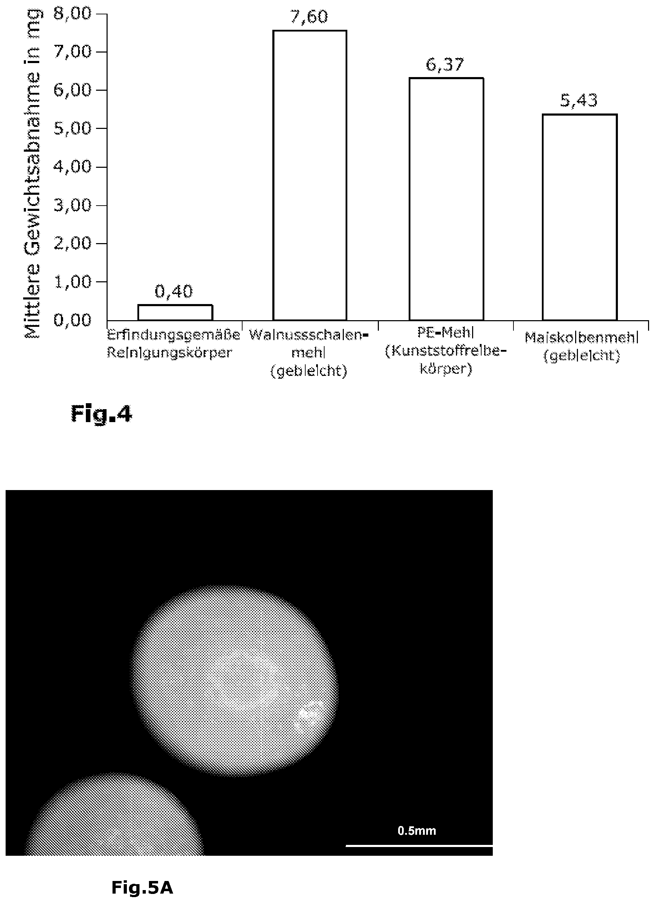

Figur 4 zeigt die Abrasionswirkung im Vergleich zu gebleichtem Wallnussschalenmehl, Polyethylenmehl und Maiskolbenmehl.

### Beispiel 7

Zum Nachweis der Schmutzbindungswirkung wurden die erfindungsgemäßen Reibkörper mit einem Normschmutz:

| | |
|---|---|
| 54,00% | Paraffinum liquid |
| 18,10% | Vaseline |
| 3,60% | Graphit |
| 18,10% | Wollwachs |
| 5,40% | Flammruß |
| 0,80% | Eisen (III) oxid |

versetzt und anschließend in einem Sieb mit Wasser gespült.

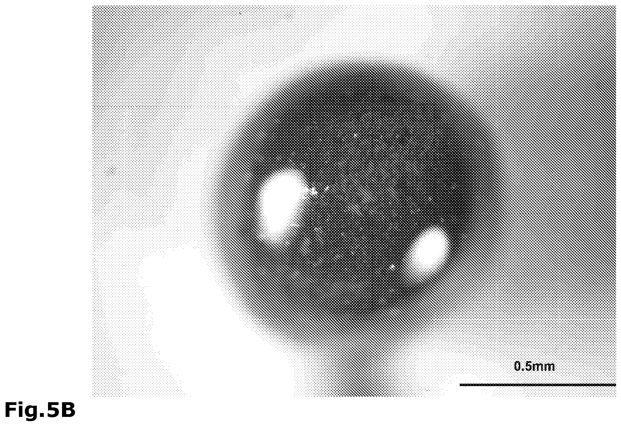

Die Figur 5a zeigt die Reinigungskörper vor der Behandlung, Figur 5b nach der Behandlung. Die Bindung des Schmutzes ist deutlich zu erkennen.

### Beispiel 8

In einem Hautverträglichkeitstest wurde die Wirkung auf die Haut bei siebentägiger Anwendung untersucht. Untersucht wurde die Wirkung einer Behandlung mit
a) Wasser,
b) üblichem Spülmittel,
c) erfindungsgemäße Reinigungskörper im Spülmittel gemäß b) suspendiert,
d) mit gebleichtem Wallnussschalenmehl im Spülmittel gemäß b) suspendiert.

Mit dem entsprechenden Behandlungsmittel wurde täglich gewaschen. Dabei wurde die Wirkung sowohl bei einer Gruppe von Atopikern als auch bei der Gruppen von Nicht-Atopikern untersucht.

Dabei zeigte sich, dass der TEWL-Wert (Transepidermaler Wasserverlust) in der Gruppe der Atopiker abnimmt. Der TEWL nimmt mit der Behandlung c) von 6 auf 3,2 ab, während er bei Einsatz von Behandlung d) auf 6,4 steigt.

In der Corneometrie nimmt der Feuchtigkeitsgehalt der Haut in der Atopiker Gruppe zu, bei der Behandlung mit Walnussschalenmehl ab.

Die Hautrötung wird bei allen Gruppen durch die Behandlung leicht verringert. Bei pH-Wert, Hautfett und Hautelastizität zeigen sich keine relevanten Unterschiede.

## Patentansprüche

1. Hautreinigungsmittel enthaltend
- 2 bis 25 Gew.-% Reinigungskörper mit einer mittleren Korngröße von 100 bis 1.000 µm enthaltend mindestens 50% hydriertes Rizinusöl, wobei die Reinigungskörper kein Natrium-Kupferchlorophylin enthalten
- 2 bis 30 Gew.-% Tenside,
- 0,1 bis 10 Gew.-% Verdickungsmittel,
- Wasser, sowie gegebenenfalls weiteren Hilfsstoffe.

2. Hautreinigungsmittel nach Anspruch 1, **dadurch gekennzeichnet, dass** das Rizinusöl vollständig oder partiell hydriert ist.

3. Hautreinigungsmittel nach einem der Ansprüche 1 oder 2, **dadurch gekennzeichnet, dass** die Reinigungskörper zusätzlich Paraffinwachse, Carnaubawachse, Candelilawachse, Polyethylenwachse, oxidierte Polyethylenwachse oder Mischungen davon mit einem Schmelzpunkt oberhalb von 40°C enthalten.

4. Hautreinigungsmittel nach Anspruch 1, **dadurch gekennzeichnet, dass** das Verdickungsmittel aus Bentoniten, Xanthanen, Acrylaten, Alginate, Celluloseether, Carrageen und Mischungen davon ausgewählt wird.

5. Hautreinigungsmittel nach mindestens einem der Ansprüche 1 bis 4, **dadurch gekennzeichnet, dass** zusätzlich eine oder mehrere der folgenden Substanzen enthalten sind
- Rückfetter,
- Farbstoffe,
- Parfümstoffe,
- Titandioxid,
- Puffersubstanzen,
- Konservierungsmittel,
- flüssige Parafine,
- Glycerin,
- Antioxidationsmitteln,
- Abrasiva.

6. Verfahren zur Herstellung der Reinigungskörper mit einer mittleren Korngröße von 100 bis 1.000 µm, die mindestens 50% hydriertes Rizinusöl enthalten, wobei die Reinigungskörper kein Natrium-Kupferchlorophylin enthalten umfassend die Schritte
- des Schmelzens von hydriertem Rizinusöl gegebenenfalls zusammen mit weiteren Wachsen
- Vertropfen oder Versprühen der Schmelze.

7. Verwendung von Reinigungskörpern mit einer mittleren Korngröße von 100 bis 1.000 µm, die mindestens 50% hydriertes Rizinusöl enthalten, wobei die Reinigungskörper kein Natrium-Kupferchlorophylin enthalten zur Herstellung von Kosmetika, insbesondere Hautreinigungsmitteln

## Claims

1. A skin cleaning agent comprising
- from 2 to 25% by weight of cleaning bodies having a mean grain size of from 100 to 1000 µm and comprising at least 50% hydrogenated castor oil, wherein said cleaning bodies do not comprise any sodium copper chlorophyllin;
- from 2 to 30% by weight of surfactants;
- from 0.1 to 10% by weight of thickeners;
- water, and optionally further auxiliaries.

2. The skin cleaning agent according to claim 1, **characterized in that** said castor oil is completely or partially hydrogenated.

3. The skin cleaning agent according to either of claims 1 or 2, **characterized in that** said cleaning bodies additionally comprise paraffin waxes, carnauba waxes, candelilla waxes, polyethylene waxes, oxidized polyethylene waxes or mixtures thereof having a melting point of above 40 °C.

4. The skin cleaning agent according to claim 1, **characterized in that** said thickener is selected from bentonites, xanthan gums, acrylates, alginates, cellulose ethers, carrageenan and mixtures thereof.

5. The skin cleaning agent according to at least one of claims 1 to 4, **characterized by** additionally comprising one or more of the following substances:
- refatting agents;
- colorants;
- perfumes;
- titanium dioxide;
- buffer substances;
- preservatives;
- liquid paraffins;
- glycerol;
- antioxidants;
- abrasives.

6. A process for preparing the cleaning bodies having a mean grain size of from 100 to 1000 µm and comprising at least 50% hydrogenated castor oil, wherein said cleaning bodies do not comprise any sodium copper chlorophyllin, comprising the steps of:
- melting hydrogenated castor oil, optionally together with further waxes;
- dispersing the melt into droplets or spraying it.

7. Use of cleaning bodies having a mean grain size of from 100 to 1000 µm and comprising at least 50% hydrogenated castor oil, wherein said cleaning bodies do not comprise any sodium copper chlorophyllin, for the preparation of cosmetics, especially skin cleaning agents.

## Revendications

1. Produit de nettoyage de la peau qui contient :
- 2 à 25 % en poids en corps de nettoyage dont la taille de grain moyenne est comprise entre 100 et 1 000 µm et contenant au moins 50 % d'huile de ricin hydrogénée, les corps de nettoyage ne contenant aucun complexe de chlorophyline cuivrique de sodium
- 2 à 30 % en poids en agents tensioactifs,
- 0,1 à 10 % en poids en agent épaississant,
- de l'eau, ainsi que d'autres adjuvants, le cas échéant.

2. Produit de nettoyage de la peau selon la revendication 1, **caractérisé en ce que** l'huile de ricin est hydrogénée en partie ou en intégralité.

3. Produit de nettoyage de la peau selon l'une des revendications 1 ou 2, **caractérisé en ce que** les corps de nettoyage contiennent en outre des cires de paraffine, des cires de carnauba, des cires de candelilla, des cires de polyéthylène, des cires de polyéthylène oxydées ou des mélanges de celles-ci, avec un point de fusion supérieur à 40 °C.

4. Produit de nettoyage de la peau selon la revendication 1, **caractérisé en ce que** l'agent épaississant est sélectionné à partir des bentonites, des gommes de xanthane, des acrylates, des alginates, de l'éther de cellulose, des carraghénanes et des mélanges de ceux-ci.

5. Produit de nettoyage de la peau selon au moins l'une des revendications 1 à 4, **caractérisé en ce que** ledit produit de nettoyage de la peau contient en outre une ou plusieurs des substances suivantes :
- des agents regraissants,
- des colorants,
- des substances parfumantes,
- du dioxyde de titane,
- des substances tampons,
- des agents conservateurs,
- de la paraffine liquide,
- de la glycérine,
- des agents antioxydants,
- des agents abrasifs.

6. Procédé destiné à la fabrication de corps de nettoyage dont la taille de grain moyenne est comprise entre 100 et 1 000 µm et contenant au moins 50 % d'huile de ricin hydrogénée, les corps de nettoyage ne contenant aucun complexe de chlorophyline cuivrique de sodium, lequel procédé comprend les phases suivantes :
- la fusion de l'huile de ricin hydrogénée, combinée le cas échéant avec d'autres cires,
la projection en goutte-à-goutte ou la pulvérisation de la masse fondue.

7. Utilisation de corps de nettoyage dont la taille de grain moyenne est comprise entre 100 et 1 000 µm et contenant au moins 50 % d'huile de ricin hydrogénée, les corps de nettoyage ne contenant aucun complexe de chlorophyline cuivrique de sodium, laquelle utilisation est destinée à la fabrication de produits cosmétiques, en particulier de produits de nettoyage de la peau.
